# EUROPEAN PATENT APPLICATION

(11) **EP 2 832 296 A1**
(43) Date of publication of application: **04.02.2015**
(21) Application number: 13767736.5
(22) Date of filing: 24.01.2013
(51) Int. Cl.: A61B 8/00

(54) **PORTABLE ULTRASONIC DIAGNOSIS DEVICE**

(30) Priority: 28.03.2012 JP 2012074315
(71) Applicant: Hitachi Aloka Medical, Ltd., Tokyo 181-8622 (JP)
(72) Inventor: NINOMIYA, Atsushi, Tokyo 100-8280 (JP); YANASE, Kazuyuki, Tokyo 100-8280 (JP); YOKOYAMA, Masaru, Tokyo 100-8280 (JP); KASANAMI, Tsuneo, Mitaka-shi Tokyo 181-8622 (JP); USAMI, Katsumi, Tokyo 100-8280 (JP)
(74) Representative: Strehl Schübel-Hopf & Partner
(86) International application number: PCT/JP2013/051448
(87) International publication number: WO 2013/145825

(57) **Abstract**

A notebook type ultrasonic diagnosis device in which a display housing including a display panel is independently movable from a main body housing including an operation panel, and both of the display housing and the main body housing are integrally operated is provided. The ultrasonic diagnosis device includes two connectable and separable housings, a display housing 10 including a display panel, and an operation housing 20 including an operation panel, and handle portions 41 and 42 are provided in the housings, respectively. The handle portions of the respective housings function as a connection portion for connecting the two housings, and configure one handle by being linked together at the time of overlapping the two housings to be connected. In addition, the handle portion 41 disposed in the display housing 10 is rotatably attached to the display housing 10, and functions as a supporting base when the display housing 10 is separated from the operation housing 20 to be independently installed, and as a handle when an operator holds the display housing 10.

## Description

### Technical Field

The present invention relates to a portable ultrasonic diagnosis device, and in particular, relates to a portable ultrasonic diagnosis device in which a display device and a main body are separable from each other.

### Background Art

An ultrasonic diagnosis device has been widely spread as a medical image diagnosis device which is relatively simple and non-invasive, and a small or a portable ultrasonic diagnosis device which is able to be used in a patient's room inside a medical center (hospital) or outside the medical center as well as in an examination room inside the medical center has been variously developed. For example, a notebook type ultrasonic diagnosis device including a structure in which a display panel for displaying an image captured by the ultrasonic diagnosis device or the like, and an operation panel for performing an input manipulation of an instruction necessary for an ultrasonic measurement are connected to be openable and closable in one end is practically used.

In the notebook type ultrasonic diagnosis device, a structure in which a display housing including the display panel is rotatable with respect to a main body housing has been proposed (for example, Patent document 1). In such a type of ultrasonic diagnosis device, it is possible to rotate the display panel in a direction of 360 degrees by rotating the display panel in a state where the display panel is erected with respect to the main body.

In addition, as a structure in which a position of the display panel is changeable, a device in which the display housing and the main body housing are able to be changed from a position (an open mode) where they are opened or closed by a hinge to a position (a tablet mode) where the display panel overlaps on the main body housing has been proposed (Patent document 2). In a technology disclosed in PTL 2, at the time of the examination, the examination is performed while performing a manipulation by the operation panel in the open mode, and when the image of the display panel is observed by a plurality of people, the image is able to be viewed in a state where the display panel overlaps with the main body housing.

### Prior Art Document

### Patent Document

[Patent document 1] JP-A-2010-162107
[Patent document 2] JP-A-2011-5241

### Summary of Invention

### Technical Problem

The ultrasonic diagnosis device disclosed in Patent document 1 adopts the structure in which the display panel is rotatable, and thus a range in which the display panel is able to be viewed is widened. However, a rotation operation is performed by connecting the display panel onto an arm protruding from the operation panel, and thus, for example, it may be difficult to perform the rotation operation in the narrow space of a bedside, and there is a limit to degrees of freedom in a movable range of the display panel.

In addition, a technology disclosed in Patent document 2, the position of the display panel is switched between two modes, and thus the manipulation of the operation panel is not able to be performed in the state where the display panel overlaps with the main body housing, and only visual recognition of the display panel is performed. That is, it is not possible to perform the manipulation of the operation panel while securing degrees of freedom of viewing the display panel.

The present invention is to provide a portable ultrasonic diagnosis device in which a display housing including a display panel is independently movable from a main body housing including an operation panel, and both of the display housing and the main body housing are integrally operated.

### Solution to Problem

In order to solve the problem described above, the present invention provides a portable ultrasonic diagnosis device including a first housing which includes a display panel, and a second housing which includes an operation panel, in which the first housing and the second housing are able to be separated from each other, and a handle is formed by linking the first housing and the second housing together. Advantageous Effects of Invention

According to the present invention, the display housing including the display panel is able to be separated from the main body housing, and thus the display panel is able to display an image or the like in any position or in a state of being held, and it is possible to remarkably improve flexibility in a range of viewing the display panel.

### Brief Description of Drawings

Fig. 1 is an entire perspective view of one embodiment of an ultrasonic diagnosis device according to the present invention.
Fig. 2 is a perspective view illustrating a different state (an opened state) of the ultrasonic diagnosis device of Fig. 1.
Fig. 3 is a diagram illustrating one embodiment of a function configuration of the ultrasonic diagnosis device according to the present invention.
Fig. 4 is a perspective view when the ultrasonic diagnosis device of Fig. 1 is viewed from a back surface side.
Fig. 5 is a perspective view when a state in which a display housing and an operation housing are separated from each other is viewed from a front surface side.
Fig. 6 is a perspective view when the state in which the display housing and the operation housing are separated from each other is viewed from the back surface side.
Fig. 7 is a top view illustrating the state in which the display housing and the operation housing are separated from each other.
Figs. 8A to 8C are diagrams illustrating a handle portion of the display housing, in which Fig. 8A is a perspective view when the handle portion is viewed from a first surface side, Fig. 8B is a perspective view when the handle portion is viewed from a second surface side, and Fig. 8C is a side view thereof.
Figs. 9A and 9B are diagrams for describing a connection between the display housing and the operation housing.
Figs. 10A and 10B are side views illustrating a posture of the display housing and the operation housing in a connected state, respectively.
Fig. 11 is a diagram illustrating a carried posture of the display housing and the operation housing in the connected state.
Figs. 12A and 12B are side views illustrating a posture of the display housing separated from the operation housing, respectively.
Fig. 13 is a perspective view illustrating a used state of the display housing separated from the operation housing.
Fig. 14 is a perspective view illustrating a different used state of the display housing separated from the operation housing.

### Description of Embodiments

A portable ultrasonic diagnosis device of the present invention includes connectable and separable two housings as basic elements. One of the two housings is provided with a display panel, the other is provided with an operation panel, and the respective housings include handle portions. The handle portions of the respective housings have a function as a connection portion which connects the two housings, are disposed in a position where the handle portions overlap with each other at the time of overlapping the two housings to be connected, and the handle portions are integrated into one handle. In addition, the handle portion disposed in the housing provided with the display panel is rotatably attached to the housing, and functions as a supporting base at the time when this housing is separated from the other housing and installed independently, and as the handle at the time of being carried by an operator.

Hereinafter, embodiments of the portable ultrasonic diagnosis device (hereinafter, simply referred to as an "ultrasonic diagnosis device") of the present invention will be described with reference to the drawings.

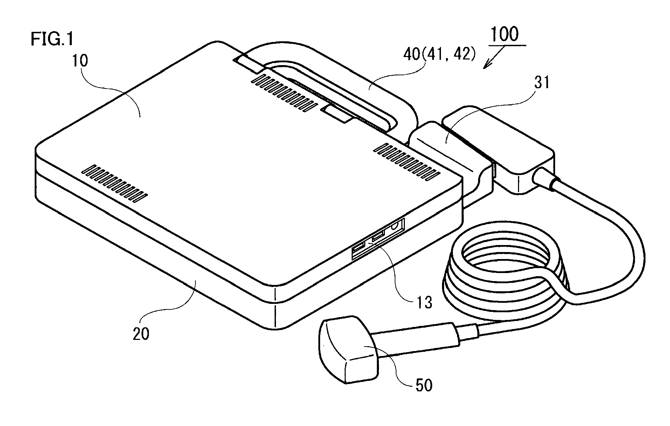

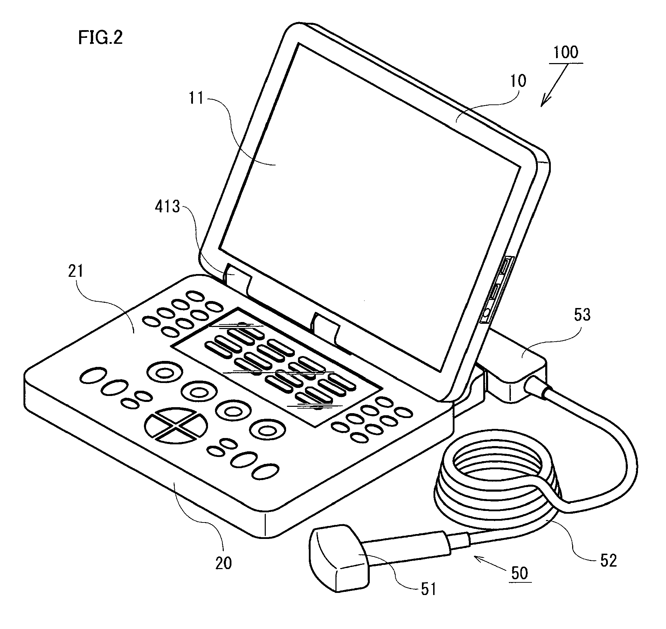

Fig. 1 and Fig. 2 are perspective views illustrating the entire ultrasonic diagnosis device of this embodiment, Fig. 1 illustrates a state in which the display panel is closed, and Fig. 2 illustrates a state in which the display panel is opened. In the following description, from a view point of the operator facing the opened display panel, the operator side of the device is a front surface, and the opposite side thereof is a back surface. In addition, the back surface side toward the front surface is a backward direction.

The ultrasonic diagnosis device 100 includes a first housing (a display housing) 10 provided with a display panel 11, a second housing (an operation housing) 20 provided with an operation panel 21, and a handle 40 as a main configuration. Devices for realizing functions necessary for the ultrasonic measurement which are able to be shared by the two housings or provided in any one or both of the housings are housed in the respective housings. The handle 40 is formed by linking the two housings 10 and 20 together in a linked state, and the display panel 11 of the first housing 10 and the operation panel 21 of the second housing 20 face each other.

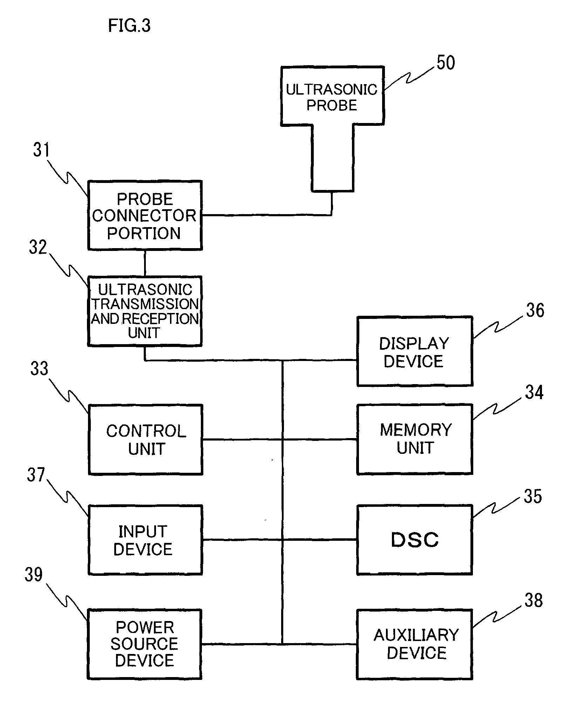

Specifically, devices necessary for the ultrasonic measurement include an ultrasonic probe (a probe) 50, a connector portion 31 to which the probe 50 is connected, an ultrasonic transmission and reception unit 32, a control unit 33, a memory unit 34, a DSC 35, a display device 36, an input device 37, an auxiliary device 38, a power source device 39, and the like as illustrated in Fig. 3. The probe 50 is a probe selected according to a target or a purpose of an examination from various types of probes and connected to the connector portion 31 disposed in the housing.

The display device 36 includes the display panel and a driving device of the display panel, and is provided in the display housing 10 in this embodiment. However, a small display panel and a driving device thereof are able to be provided in the operation housing 20. The input device 37 includes an input key or a button, a GUI, and the like which input instructions necessary for operating the device, and is provided in the operation housing 20 as the operation panel 21 in which the input key or the button, the GUI, and the like are suitably arranged. However, an input device such as a touch panel is able to be provided in the display housing 10. The power source device 39 and a switch (not illustrated) for turning the power source device ON or OFF are provided in both of the display housing 10 and the operation housing 20. The power source device 39 is a simple power source such as a battery, and may be provided with a terminal for supplying electric power from an external power source. For example, the other devices 31 to 35, and 38 are able to be provided in one or both of the display housing 10 and the operation housing 20. In the ultrasonic diagnosis device 100 of this embodiment, as main components, the devices 31 to 35, and 38 are provided in the operation housing 20, and the memory unit 34 for the display housing is provided in the display housing 10. However, it is possible to assemble a simple device having the same function as the devices 31 to 35 in the display housing 10.

In the operation housing 20 and the display housing 10, a mechanical connection portion (described later) also serves as an electrical connection portion, and a data exchange between the devices in the housings may be performed through the electrical connection portion. However, the data exchange is able to be performed by wired or wireless connection. For this reason, the display housing 10 may include an input and output terminal 13 such as a USB port for performing the data exchange with devices in the operation housing 20, other ultrasonic diagnosis devices (including a device in addition to the portable device) or the like. Further, when the display housing 10 has an ultrasonic transmission and reception function, the display housing 10 may include the connector portion 31 of the probe 50. Similarly, the operation housing 20 may include an input and output terminal (not illustrated) for performing the data exchange with devices in the display housing 10, other ultrasonic diagnosis devices, an external display device, or the like.

Next, a structure of the ultrasonic diagnosis device will be described. In this embodiment, the display housing 10 and the operation housing 20 have thin thicknesses, and are formed in the shape of a rectangular parallelepiped in which corner portions of a side surface are rounded, respectively. In addition, a surface (a display surface) on which the display panel 11 of the display housing 10 is disposed, and a surface (a manipulation surface) on which the operation panel 21 of the operation housing 20 is disposed have the same shape and the same size, and thus the display housing and the operation housing are formed in the shape of an integrated rectangular parallelepiped without dislocation (deviation) on the side surface in a state where the display housing and the operation housing overlap with each other as illustrated in Fig. 1. Thus, the display housing 10 and the operation housing 20 have an external appearance of a so-called notebook type ultrasonic diagnosis device.

The display housing 10 and the operation housing 20 are connected to each other by the handle 40 on one side surface side thereof. The display housing 10 is rotatably fixed to the handle 40, and thus the display housing is able to be in a closed state illustrated in Fig. 1 and in an opened state illustrated in Fig. 2 with respect to the operation housing 20. In the opened state, the display housing is able to freely change an opening angle with respect to the operation housing 20. Here, in the connection portion between the display housing 10 and the handle portion 41, resistance can be imparted to rotation of the display housing and the handle portion, and thus it is possible to fix the display housing 10 at any angle position. In addition, a locking mechanism for locking the closed state of the display housing 10 may be provided in positions of the display housing 10 and the operation housing 20 which face each other. As the locking mechanism, a known locking mechanism including a combination of a hook and a hole with which the hook is engaged, or the like is able to be adopted.

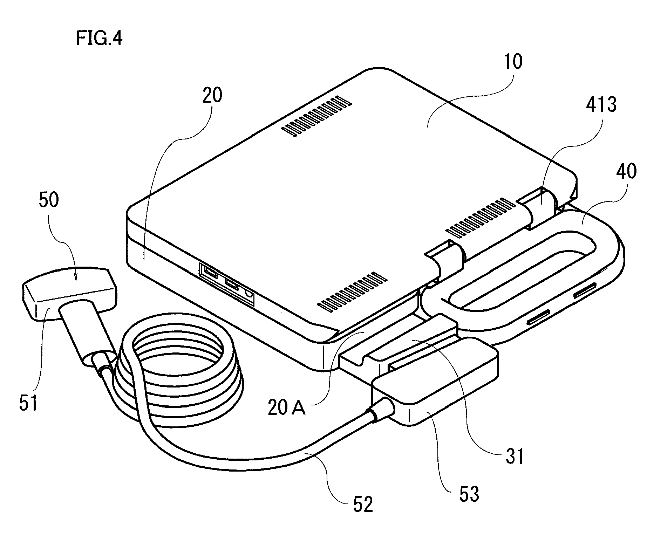

On a side surface (a back surface) 20A of the operation housing 20 on which the handle 40 is disposed, the connector portion 31 for connecting the probe 50 thereto is formed to protrude toward the backward direction along with the handle 40, as illustrated in Fig. 4. It is possible to make the operation housing 20 compact by arranging a member for configuring the connector portion 31 outside the device without housing the member in the device, and it is possible to dispose the connector portion 31 without impairing the appearance of the entire device by using a space on the side of the handle 40 and in the backward direction. Furthermore, the probe 50 includes a probe main body 51 in which a plurality of ultrasonic transducers are arranged, a cable 52, and a socket portion 53 which is connected to the connector 31. Various types of probes in which the probe main bodies have different shapes corresponding to a measurement portion are prepared, and the examination is performed by connecting a probe selected according to the purpose of the examination to the connector 31.

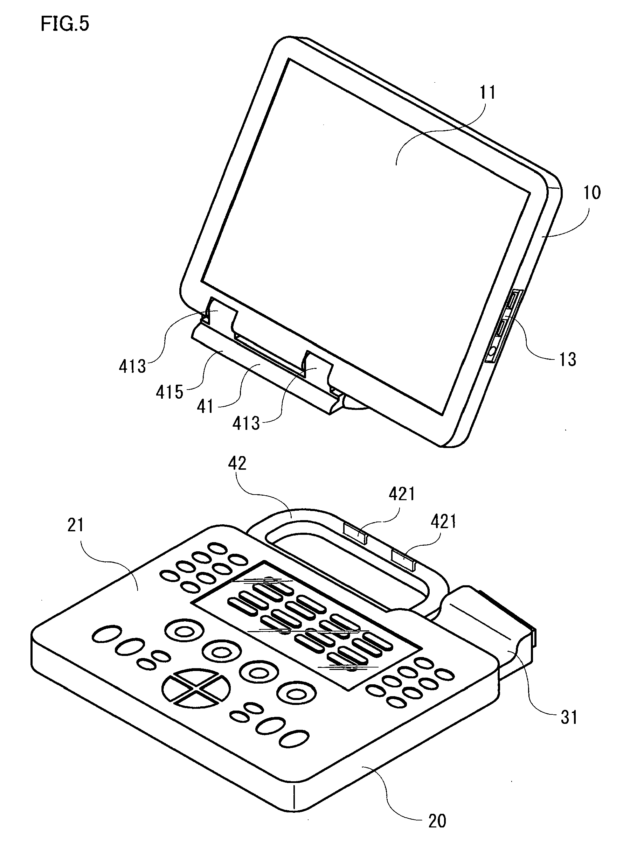

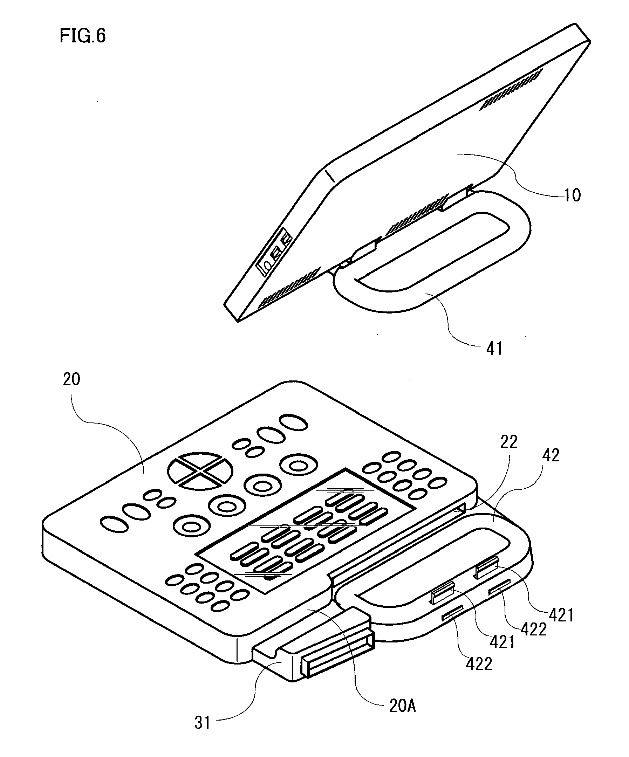

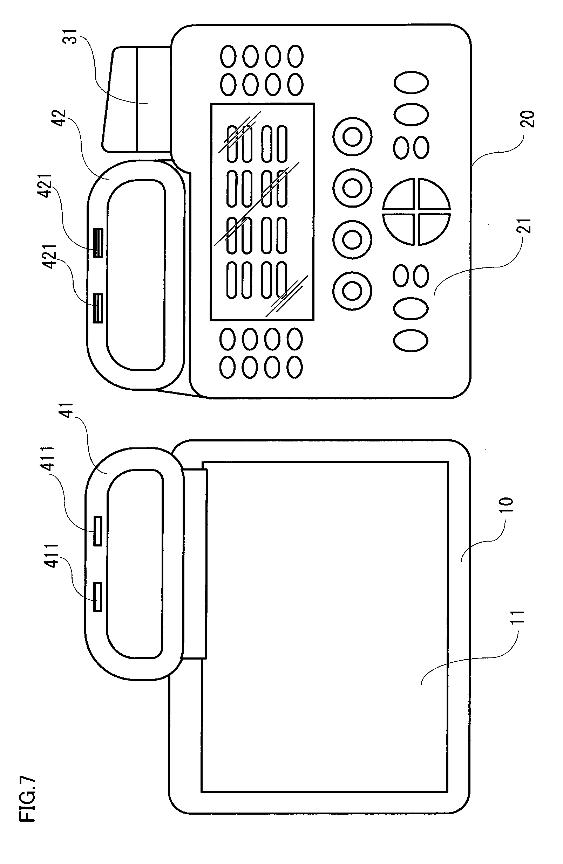

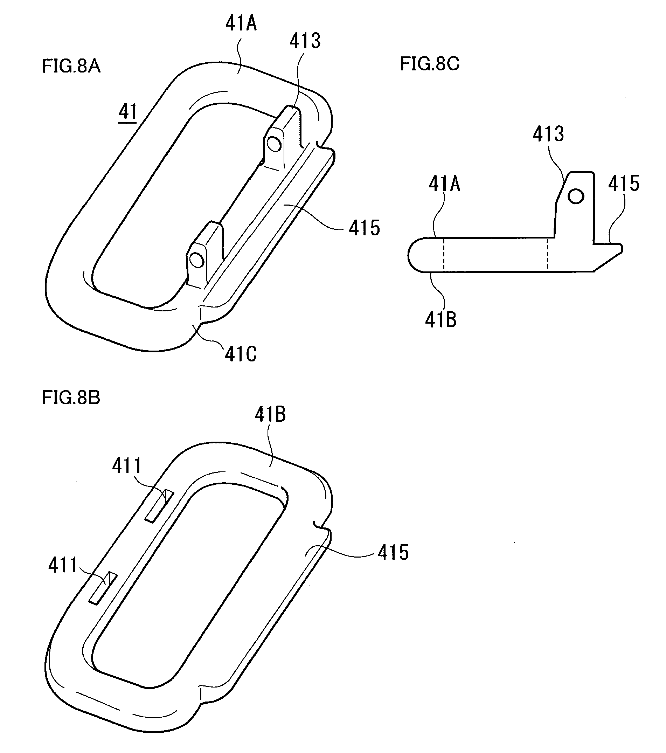

Next, a mechanism for separating and linking (coupling) the display housing 10 and the operation housing 20 will be described with reference to Fig. 5 to Fig. 8B. Fig. 5 and Fig. 6 are perspective views illustrating a state in which the ultrasonic diagnosis device of this embodiment is separated into the two housings, respectively, Fig. 5 is a diagram when viewed from the front surface, and Fig. 6 is a diagram when viewed from the back surface. Fig. 7 is a top view of the display housing and the operation housing at the time of being separated from each other, and Figs. 8A and 8B are perspective views illustrating the handle on the display housing side.

In the ultrasonic diagnosis device of this embodiment, the handle 40 is divided into a portion (a first handle portion 41) on the display housing 10 side, and a portion (a second handle portion 42) on the operation housing 20 side, and in the linked state illustrated in Fig. 1 and Fig. 2, both of the handle portions 41 and 42 function as the connection portion for mechanically connecting both of the housings by linking the housings together to configure one handle 40, and provide a shaft of the rotation of the display housing 10 with respect to the operation housing 20. The display housing 10 and the operation housing 20 are able to be physically separated from each other to be physically independent housings by separating the handle 40 into the two portions. In a separated state, the display housing and the operation housing are able to be used as one ultrasonic diagnosis device by wired or wireless electrical connection. In addition, it is possible to independently use each of the housings or to use the housings by connecting to a different ultrasonic diagnosis device or a display device according to the functions included in the respective housings.

The handle 40 of this embodiment has a ring shape surrounding a central elongated opening into which fingers of the operator are inserted, and the respective handle portions 41 and 42 have a shape in which the handle 40 having such a shape is divided into two parts along a direction parallel to a main plane, as illustrated in Fig. 4. An outline of a cross-section (a cross-section perpendicular to the main plane, the same applies to the following) of the ring-shaped handle portion 41 is in the shape of a rounded square or a circle. On the other hand, a cross-section of the ring-shaped handle portion 42 has a concave shape for receiving the handle portion 41. Then, when both of the handle portions 41 and 42 are connected, a cross-section of the ring-shaped handle 40 is in the shape of a rounded square or a circle as a whole.

In the handle portion 41 on the display housing 10 side, as illustrated in Fig. 5 and Fig. 6, arms 413 to which the display housing 10 is connected, and a linkage projection portion 415 for linking the display housing 10 and the operation housing 20 together are formed on one end of the ring-shaped handle portion. Structures and functions of the arms 413 and the linkage projection portion 415 will be described later. The display housing 10 is connected to the handle portion 41 through a shaft (not illustrated) passing through the arms 413.

As illustrated in Fig. 6 and Fig. 7, the handle portion 42 on the operation housing 20 side is formed integrally with an exterior case for configuring the operation housing 20 on the back surface 20A side of the operation housing 20, and a thickness of the handle portion 42 is thinner than that of the operation housing 20, and thus a step (difference in level) is provided between an operation panel surface and a top surface of the handle portion 42 (a surface in the same direction as the operation panel surface) . The top surface of the handle portion 42 is formed to have a concave shape for receiving one half of the handle portion 41 on the display housing side in a thickness direction, and a half of the handle portion 41 is fitted into a concave portion thereof.

In the handle portion 41 and the handle portion 42, locking mechanisms for connecting both of the handle portions are provided. In this embodiment, the locking mechanism includes a combination of hooks 421 and holes 411, and lock release buttons 422, and is disposed on each end portion opposite to the portion connecting with the housings. The hook 421 which is erectly disposed on the top surface of the handle portion 42 is engaged with the hole 411 formed in the handle portion 41, and connects the handle portion 41 with the handle portion 42. The lock release button 422 allows the hook 421 to slide inside the hole 411, and thus releases a locked state between the hook 412 and the hole 411. Two sets of hooks and holes are illustrated in the drawings, but the combination of the hook and hole is not limited to the two sets.

In a portion, which rises up with respect to the handle portion 42, on the back surface 20A of the operation housing 20, as illustrated in Fig. 6, a connection concave portion 22 into which the connection projection portion 415 provided in the handle portion 41 on the display housing 10 side is inserted is formed. In this embodiment, the connection projection portion 415 and the connection concave portion 22 mechanically connect the display housing 10 and the operation housing 20, and serve as a connection terminal for electrically connecting the devices accommodated in both of the housings. However, the electrical connection may be separately performed by wired or wireless connection, and only the mechanical connection may be performed by the connection portion.

Further, a structure of the handle portion 41 on the display housing 10 side will be described with reference to Figs. 8A and 8B. As illustrated, in the handle portion 41, the arms 413 for connecting the display housing 10 to the end portion of the ring-shaped member, and the connection projection portion 415 connected to the operation housing 20 are formed. Figs. 8A and 8B are perspective views when the handle portion 41 is viewed from both the front surface side and the back surface side, and in the following description, a surface illustrated in Fig. 8A is referred to as a first surface 41A, a surface illustrated in Fig. 8B is referred to as a second surface 41B, and a side surface joining the first surface and the second surface together is referred to as an inner circumferential surface and an outer circumferential surface. Fig. 8C is a side view.

The arms 413 for being connected with the display housing 10 are formed in a portion of the first surface 41A of the handle portion 41 on a side opposite to a portion gripped by a user along a direction perpendicular to the first surface 41A. In an illustrated example, two arms 413 are formed at an interval from each other, and the display housing 10 is pivotally supported by the arms 413. In Fig. 5, the display housing 10 which is pivotally supported by the arms 413 is illustrated. According to this configuration, the display housing 10 is able to take any angle from 0 degrees to 180 degrees with respect to the first surface 41A of the handle portion 41.

The connection projection portion 415 which is the connection portion with the operation housing 20 is formed in a portion of the outer circumferential surface 41C of the handle portion 41 on the side opposite to the portion gripped by the user. The connection projection portion 415 is connected to the connection concave portion 22 which is formed on the back surface 20A of the operation housing 20 described above. The connection projection portion 415 is a projection portion which is elongated along the outer circumferential surface 41C, and has a tapered-shape cross-section in which a thickness thereof becomes thinner in a direction protruding from the outer circumferential surface 41C side. A tapered surface of the connection projection portion 415 is inclined with respect to the second surface 41B illustrated in Fig. 8B, and thus when the connection projection portion 415 is connected to the connection concave portion 22 of the operation housing 20, it is possible to smoothly insert the connection projection portion 415 into the connection concave portion 22 in a state where the first surface 41A of the handle portion 41 is slightly inclined upward with respect to a horizontal surface, and it is possible to improve manipulability.

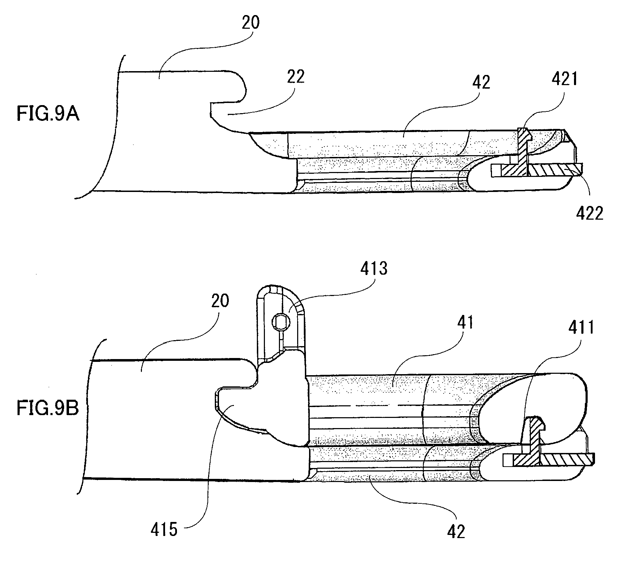

Next, connection mechanisms (411, 421, and 422) between the handle portion 42 of the operation housing 20, and the handle portion 41 of the display housing 10 will be described with reference to Figs. 9A and 9B. Fig. 9A is a side cross-sectional view illustrating the handle portion 42 of the operation housing 20, Fig. 9B is a side cross-sectional view illustrating a state in which the handle portion 41 of the display housing 10 is connected to the handle portion 42 illustrated in Fig.9A, and the display housing 10 is omitted.

When the display housing 10 and the operation housing 20 are to be connected, first, the connection projection portion 415 is inserted into the connection concave portion 22 on the back surface of the operation housing 20 in the state where the main plane of the handle portion 41 is slightly inclined from the horizontal surface, and then the handle portion 41 is made parallel to the handle portion 42. At this time, the angle of the display housing 10 connected to the handle portion 41 with respect to the handle portion 41 is arbitrary, and operability is higher in a state where the display housing 10 is slightly inclined to the handle portion 41 side compared to a position where the display housing 10 is perpendicular to the handle portion 41 side. When the connection projection portion 415 is fitted into the connection concave portion 22, and the handle portion 41 is parallel to the handle portion 42, the hook 421 of the handle portion 42 is engaged with the hole 411 of the handle portion 41, and thus the hook and the hole are locked.

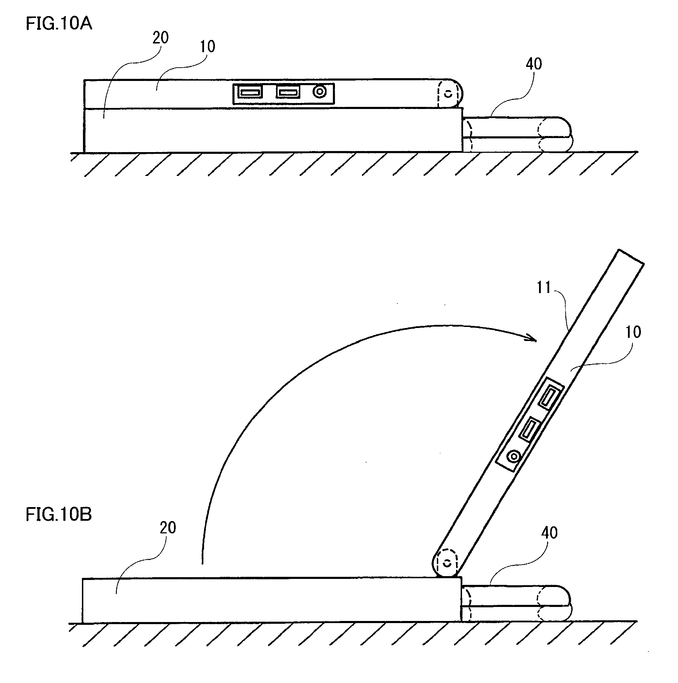

Subsequently, when the display housing 10 is rotated, and the display panel and the operation panel of the operation housing 20 are parallel to each other so as to be overlapped with each other, the display housing 10 is in the closed state illustrated in Fig. 10A. When the display housing 10 is erected at a suitable angle with respect to the operation housing 20, the display housing 10 is in the opened state as illustrated in Fig. 10B. In Figs. 10A and 10B, in order to illustrate a connected state of the handle portions 41 and 42, the connector portion is not illustrated.

On the other hand, when the display housing 10 is separated from the operation housing 20, the release button 422 is pressed towards a left direction in Fig. 9. A posture of the display housing 10 at the time of performing this manipulation is not limited, but operability is higher when the display housing 10 is in the state of Fig. 10B. The hook 421 slides inside the hole interlocking with the release button 422, and thus is moved to a position which is detached from the hole. In this state, when the handle portion 41 is lifted up in a direction in which the connection projection portion 415 is retreated, the display housing 10 is separated from the operation housing 20.

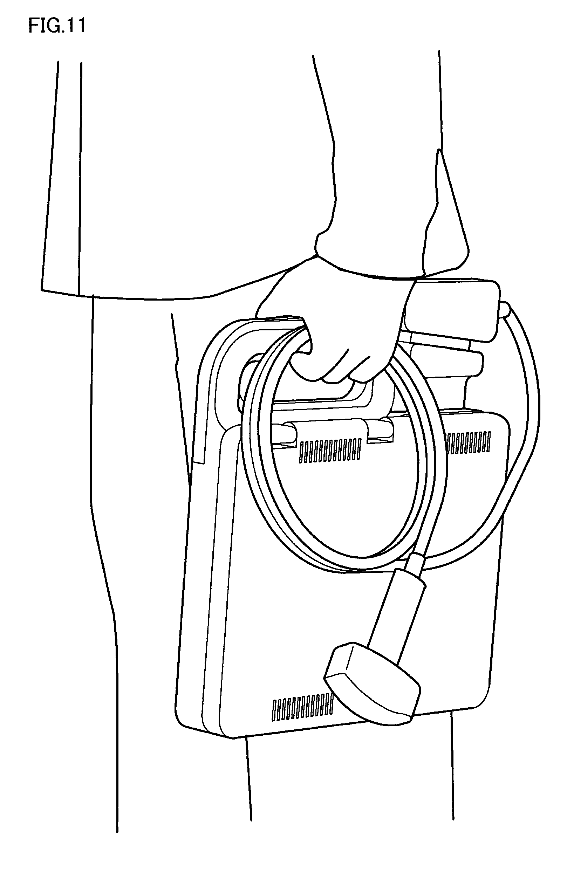

In the state where the display housing 10 and the operation housing 20 are connected, the ultrasonic diagnosis device of this embodiment is used similarly to the usual notebook type ultrasonic diagnosis device, that is, the probe 50 is connected to the connector portion 31 to perform the examination, and an obtained image is displayed on the display panel. In addition, as illustrated in Fig. 11, it is possible to carry the device by the handle 40 combined with the two handle portions 41 and 42.

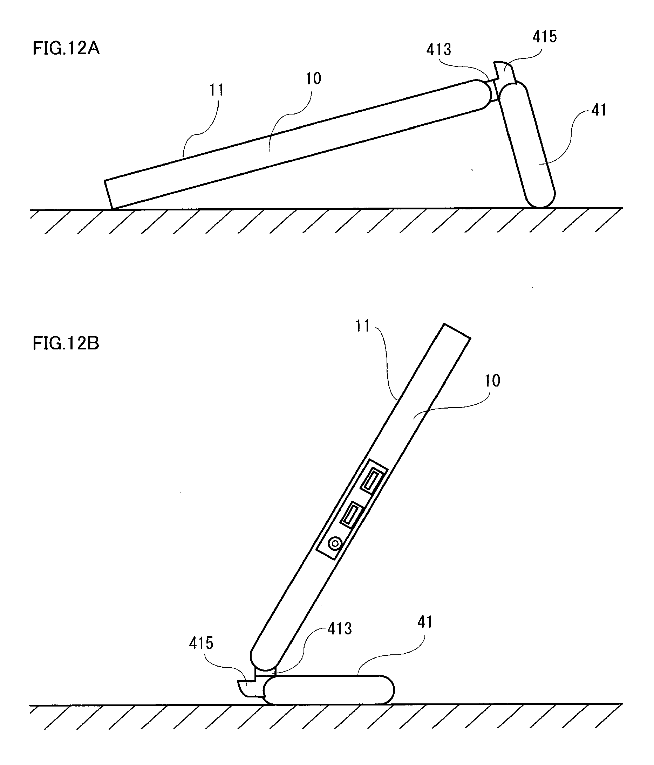

In the separated state, various usage patterns as described in the following can be adopted. A usage example of the display housing 10 in the separated state is illustrated in Fig. 12A to Fig. 14.

Figs. 12A and 12B are diagrams illustrating a state where the display housing 10 is used by being placed on a table or a stand, and a direction of the display panel is upside down in Figs. 12A and 12B. An arrangement illustrated in Fig. 12A is suitable when an eye position of a display viewer is relatively high, and the respective end portions of the handle portion 41 and the display housing 10 support the entire device such that the connection portion between the handle portion 41 and the display housing 10 is positioned on an upper side. In an arrangement illustrated in Fig. 12B, the display housing 10 is supported by the handle portion 41 as a support portion, and the arrangement is suitable when a space for placing the display housing 10 is narrow.

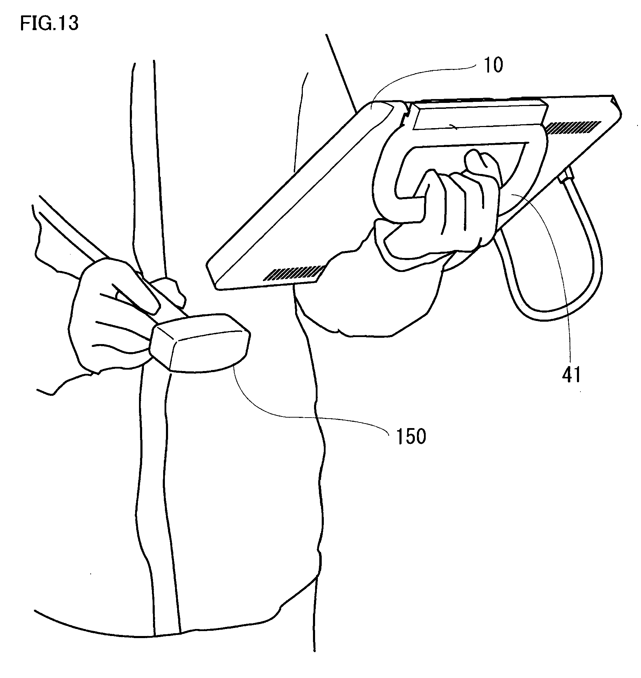

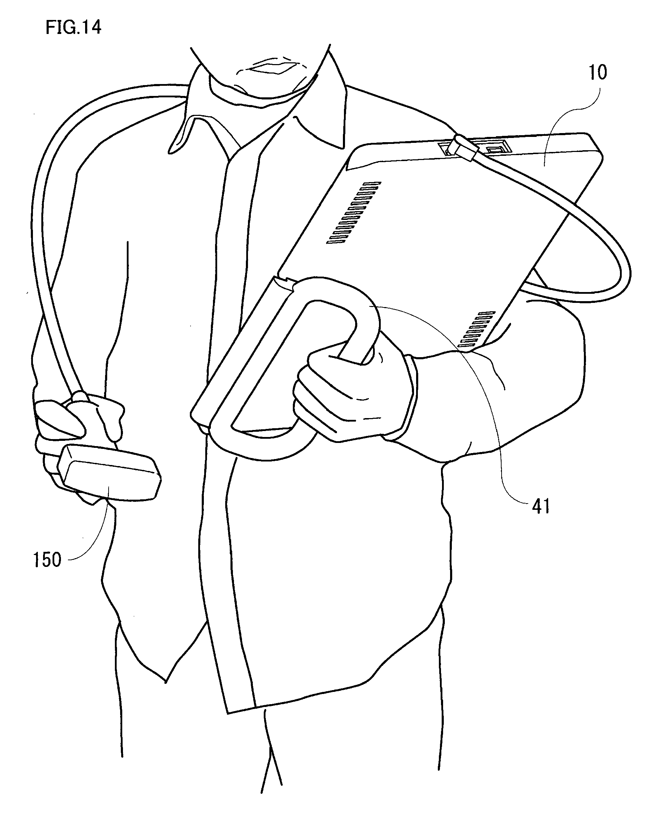

Fig. 13 and Fig. 14 are diagrams illustrating a case where the operator manipulates the display housing 10 by holding the display housing 10 with a hand, and in Fig. 13 and Fig. 14, a right and left direction and an up and down direction of the display panel are dislocated by 90 degrees. In a state illustrated in Fig. 13, the operator holds the handle portion 41 with the hand such that a longitudinal direction of the display housing 10 is the right and left direction, and thus it is possible to perform an operation by placing the display housing 10 between the hand and an elbow. In a state illustrated in Fig. 14, the operator holds the handle portion 41 such that the longitudinal direction of the display housing 10 is the up and down direction, and thus it is possible to perform the operation by supporting the display housing 10 between the hand and the elbow.

Furthermore, in an example illustrated in Fig. 13 and Fig. 14, a case where a part or an entirety of the devices (for example, the ultrasonic transmission and reception unit 32, the control unit 33, the memory unit 34, and the DSC 35 of Fig. 1) necessary for an ultrasonic examination is assembled in the display housing 10 itself is illustrated, and thus it is possible to perform a simple examination only by the display housing 10 and the devices housed in the display housing 10. Alternatively, a configuration in which the ultrasonic transmission and reception unit 32 is embedded in the probe 150 may be included. Furthermore, a configuration in which the probe 150 and the display housing 10 are connected wirelessly may be included.

In addition, the operation housing 20 is able to be connected to a display device different from the display housing 10, for example, a display with a large screen installed in a patient's room, and thus it is possible to view the image by the display while performing the examination. Alternatively, the operation housing 20 is able to be connected to another ultrasonic diagnosis device, and thus it is possible to be operated as the operation panel of the device.

According to this embodiment, the display device and the main body including the manipulation portion of the ultrasonic diagnosis device are able to be separated from each other, and thus flexibility in the posture of the display panel is remarkably improved. In addition, the display device and the main body are connected by a pair of handle portions which configure one handle, and thus it is convenient to carry or handle the device even when the device is in the linked state or in the separated state.

According to this embodiment, the display device is rotatably connected to the handle portion, and thus the display device is able to take various postures.

For example, the angle of the handle portion provided in the display panel is able to be freely changed with respect to the display housing, and thus it is possible to place the handle portion on an installation plane as a support of the display panel. At this time, it is possible to arbitrarily change the angle of the display panel with respect to the installation plane.

Further, the handle portions which are disposed in the display housing and the main body housing, respectively, are linked together to configure the handle of the device, and thus a belt or a handle for carrying the device is not necessary to be separately provided, and operability at the time of carrying the device is improved. In addition, the handle portion itself of each of the housings functions as the handle of each of the housings, and thus it is possible to independently carry each of the housings and to manipulate the device in a state of holding the handle portion, and it is possible to remarkably improve operability of the ultrasonic diagnosis device even in a narrow space.

Furthermore, the illustrated shape of the ultrasonic diagnosis device of this embodiment is an example, and the shape is able to be arbitrarily changed insofar as the shape has the functions described above. For example, in the illustrated embodiment, a case where both of the display housing and the operation housing are in the shape of a thin square and the main planes thereof have approximately the same size is illustrated, but each of the display housing and the operation housing may have any shape, and the sizes of the main planes may be different from each other. Similarly, the shape of the handle may also be a C shape or a trapezoid shape in which one side is opened as well as the ring shape insofar as the shape has a function as the handle, and the shape of the two handle portions may also be any shape insofar as the shape has a function of enabling the two handle portions to be connected with each other and functions as the handle at the time of being connected.

Further, in this embodiment, as a suitable arrangement, a case where the connector portion of the probe is arranged alongside the handle on the back surface side of the operation housing is illustrated, but the arrangement is not limited to the back surface insofar as the position of the connector portion does not interfere with the manipulation.

The following is a summary of the characteristics of the portable ultrasonic diagnosis device according to the present invention.

A first housing (a display housing) including a display panel, a second housing (an operation housing) including an operation panel surface on which an operation panel is arranged, and a first handle portion and a second handle portion for configuring one handle by being linked together are provided, the second handle portion is fixed to a main body housing, and the first handle portion is rotatably fixed to the display housing.

The display panel of the display housing and the operation panel of the operation housing face each other in a state in which the first handle portion and the second handle portion are connected.

The main body housing has a side surface perpendicular to the operation panel surface, and the second handle portion is fixed to the side surface perpendicular to the operation panel surface.

The first handle portion is provided with a projection portion for being connected to the main body housing. A concave portion with which a protruding portion of the first handle portion is engaged is formed on the side surface of the main body housing to which the second handle portion is fixed. The projection portion of the first handle portion and the concave portion of the main body housing are able to serve as a portion of electrical connection between electronic components housed in the display housing and electronic components housed in the main body housing.

The main body housing has a connector portion for connecting a probe, and the connector portion is able to be disposed on the side surface to which the second handle portion is fixed in proximity to the second handle portion.

The first handle portion and the second handle portion have a connection mechanism for engaging the first handle portion and the second handle portion with each other to be attachable and detachable.

Shapes of top surfaces of a portion excluding the first handle portion of the display housing, and a portion excluding the second handle portion of the main body housing are approximately identical to each other. In addition, the top surfaces of the portion excluding the first handle portion of the display housing, and the portion excluding the second handle portion of the main body housing are in the shape of a rectangle, and the first handle portion and the second handle portion are disposed in corresponding positions along a long side of each rectangle, respectively. In this case, the display panel of the display housing has a display mode in which a longitudinal direction of the rectangle is a top and bottom, and another display mode in which a short direction is a top and bottom.

### Explanation of Reference Signs

- 10: display housing (first housing)
- 11: display panel
- 13: input and output terminal
- 20: operation housing (second housing)
- 21: operation panel
- 22: connection concave portion
- 31: connector portion
- 40: handle
- 41: handle portion (first handle portion)
- 42: handle portion (second handle portion)
- 413: arm
- 415: connection projection portion
- 421: hook
- 422: release button
- 50, 150: probe
- 100: portable ultrasonic diagnosis device

## Claims

1. A portable ultrasonic diagnosis device, comprising:
a first housing which includes a display panel; and
a second housing which includes an operation panel,
wherein the first housing and the second housing are able to be separated from each other, and
a handle is formed by linking the first housing and the second housing together.

2. The portable ultrasonic diagnosis device according to Claim 1,
wherein a first handle portion for configuring the handle is rotatably fixed to the first housing.

3. The portable ultrasonic diagnosis device according to Claim 1,
wherein a second handle portion for configuring the handle is fixed to the second housing.

4. The portable ultrasonic diagnosis device according to Claim 1,
wherein the display panel of the first housing and the operation panel of the second housing face each other in a state in which the first housing and the second housing are linked together by the handle.

5. The portable ultrasonic diagnosis device according to Claim 3,
wherein the second housing includes a side surface perpendicular to a surface of the operation panel, and
the second handle portion is fixed to the side surface perpendicular to the surface of the operation panel.

6. The portable ultrasonic diagnosis device according to Claim 2,
wherein the first handle portion includes a projection portion for being connected to the second housing, and
a concave portion with which the projection portion of the first handle portion is engaged is formed on the side surface of the second housing.

7. The portable ultrasonic diagnosis device according to Claim 6,
wherein the projection portion of the first handle portion and the concave portion of the second housing serve as a portion of electrical connection between electronic components housed in the first housing and electronic components housed in the second housing.

8. The portable ultrasonic diagnosis device according to Claim 3,
wherein the second housing includes a connector portion for connecting a probe, and
the connector portion is arranged on the side surface to which the second handle portion is fixed in proximity to the second handle portion.

9. The portable ultrasonic diagnosis device according to Claim 1,
wherein shapes of top surfaces of the first housing and the second housing are identical to each other in portions excluding the handle.

10. The portable ultrasonic diagnosis device according to Claim 9,
wherein the top surfaces of the portion excluding the handle of the first housing and the portion excluding the handle of the second housing are in the shape of a rectangle, and
the handle is arranged on a corresponding position along a long side of each rectangle.

11. The portable ultrasonic diagnosis device according to Claim 10,
wherein the display panel of the first housing has a display mode in which a longitudinal direction of the rectangle is a top and bottom, and a display mode in which a short direction of the rectangle is a top and bottom.

12. The portable ultrasonic diagnosis device according to Claim 2,
wherein the second handle portion for configuring the handle is fixed to the second housing.

13. The portable ultrasonic diagnosis device according to Claim 12,
wherein the first handle portion and the second handle portion include a connection mechanism which engages the first handle portion and the second handle portion with each other to be attachable and detachable.

14. A portable ultrasonic diagnosis device which includes a first housing including a display panel, and a second housing including an operation panel surface on which an operation panel is arranged, comprising:
a first handle portion and a second handle portion which configure one handle by being linked together,
wherein the first handle portion is rotatably fixed to the first housing, and
the second handle portion is fixed to the second housing.

15. The portable ultrasonic diagnosis device according to Claim 14,
wherein the first handle portion and the second handle portion include a connection mechanism which engages the first handle portion and the second handle portion with each other to be attachable and detachable.
